(19)

Europäisches
Patentamt
European
Patent Office
Office européen
des brevets

(11)  **EP 3 660 169 A1**

(12)  **EUROPEAN PATENT APPLICATION**

(43) Date of publication:
  **03.06.2020  Bulletin 2020/23**

(51) Int Cl.:
  ***C12Q 1/6876*** (2018.01)

(21) Application number: **18382873.0**

(22) Date of filing: **30.11.2018**

(84) Designated Contracting States:
  **AL AT BE BG CH CY CZ DE DK EE ES FI FR GB
  GR HR HU IE IS IT LI LT LU LV MC MK MT NL NO
  PL PT RO RS SE SI SK SM TR**
  Designated Extension States:
  **BA ME**
  Designated Validation States:
  **KH MA MD TN**

(71) Applicants:
  • **Hospital Sant Joan de Deu
  08950 Esplugues de Llobregat Barcelona (ES)**
  • **Universitat Politècnica De Catalunya
  08034 Barcelona (ES)**

(72) Inventors:
  • **Castellano Escuder, Pol
  08720 VILAFRANCA DEL PENEDÈS (ES)**

  • **Maqueda González, María
  08902 HOSPITALET DE LLOBREGAT (ES)**
  • **Diaz, Ruben
  08025 BARCELONA (ES)**
  • **Ramón Krauel, Marta
  08023 BARCELONA (ES)**
  • **Lerín Martínez, Carlos
  08029 BARCELONA (ES)**
  • **Perera Lluna, Alexandre
  08028 VILANOVA I LA GELTRÚ (ES)**
  • **Jiménez Chillarón, José Carlos
  08025 BARCELONA (ES)**

(74) Representative: **ZBM Patents - Zea, Barlocci &
  Markvardsen
  Rambla Catalunya, 123
  08008 Barcelona (ES)**

(54)  **MARKERS FOR PREDICTING WEIGHT LOSS TREATMENT RESPONSE IN CHILDREN**

(57)  The present invention provides a method for predicting the responsiveness of a subject to a non-surgical weight loss intervention, which comprises the step of determining, in an isolated test sample from the subject, the methylation status of one or more genes selected from the group consisting of NRP2, GSDMD, LOC102800447, NLRC5, GFRA1, LTBP3, and SPTLC2; or alternatively the CpG position cg05387464; or alternatively one or more genes selected from the group consisting of NRP2, GSDMD, LOC102800447, NLRC5, GFRA1, LTBP3, and SPTLC2; in combination with the CpG position cg05387464. The method of the invention allows a precise prediction of the effectiveness of weight loss treatments.

EP 3 660 169 A1

## Description

## Technical Field

The present invention relates to the field of clinical treatment prognosis. In particular, the invention relates to the use of markers for predicting the response of subjects to weight loss treatments. The markers of the invention are particularly useful for the prediction of weight loss response in pre-pubertal children.

## Background Art

Genetic and lifestyle factors as well as gene-environment interactions have been involved in the etiology of obesity. In recent years, epigenetics has risen as a new tool to understand the influence of lifestyle factors on obesity. In this sense, epigenetics refers to the study of on the inheritance of patterns of gene expression that occur without changes in the DNA sequence.

One of the most studied epigenetic mechanisms in relation to obesity is DNA methylation at cytosines followed by guanines (CpG positions). The use of DNA methylation as a biomarker presents several advantages such as stability, frequency, and noninvasive accessibility in bodily fluids.

There is a growing body of evidence that shows a relevant role of these epigenetic marks in obesity and its codiseases susceptibility. Strikingly, therapeutic strategies for counteracting excess body weight are able to remodel DNA methylation profiles concomitant with the reduction of body weight.

DNA methylation status has been found to be informative of response to weight loss treatments in adult population, prior to beginning treatment. Thus, a stratification of adult subjects between low and high responders can be performed by determining the differences in the DNA methylation patterns (Milagro FI et al., "A dual epigenomic approach for the search of obesity biomarkers: DNA methylation in relation to diet-induced weight loss" FASEB J. 2011; vol. 25(4), pp. 1378-89).

These encouraging results with methylation marks in adult population cannot be extrapolated to children: The epigenome is highly dynamic during early development and childhood, giving rise to strong rearrangements of DNA methylation (Xu C.J. et al., "The emerging landscape of dynamic DNA methylation in early childhood", BMC Genomics., 2017, vol. 18(1), pp. 25).

Therefore, in spite of all the efforts made, there is still the need of reliable methods to predict the responsiveness to standard antiobesity treatments, especially in children.

## Summary of Invention

The present inventors have found a new set of methylation markers that are capable of predicting the responsiveness of subjects to non-surgical weight loss treatments.

As shown in the examples below, the inventors have found that the methylation status of CpG positions located within the genes NRP2, GSDMD, LOC102800447, NLRC5, GFRA1, LTBP3, and SPTLC2, or the methylation status of the CpG position cg05387464, located in an intergenic region, can individually predict with strong statistical power the responsiveness of subjects to weight loss interventions. Therefore, with the simple analysis of a single methylation position the method of the invention allows for a precise prediction of the effectiveness of weight loss treatments.

Thus, in a first aspect, the invention provides a method for predicting the responsiveness of a subject to a non-surgical weight loss intervention, which comprises the step of determining in an isolated test sample from the subject the methylation status of one or more genes selected from the group consisting of NRP2, GSDMD, LOC102800447, NLRC5, GFRA1, LTBP3, and SPTLC2; or alternatively the CpG position cg05387464; or alternatively one or more genes selected from the group consisting of NRP2, GSDMD, LOC102800447, NLRC5, GFRA1, LTBP3, and SPTLC2; in combination with the CpG position cg05387464.

As mentioned above, children have a high dynamic epigenome. Strikingly, the markers of the invention provide precise prognostic information in children population.

The markers of the invention are highly versatile since they can provide reliable information independently of the technique used to determine the methylation status.

In a second aspect the invention provides use of means for determining, in an isolated test sample, the methylation status of one or more genes selected from the group consisting of NRP2, GSDMD, LOC102800447, NLRC5, GFRA1, LTBP3, and SPTLC2; or alternatively the CpG position cg05387464; or alternatively one or more genes selected from the group consisting of NRP2, GSDMD, LOC102800447, NLRC5, GFRA1, LTBP3, and SPTLC2, in combination with the CpG position cg05387464; in a method for predicting the responsiveness of a subject to a weight loss intervention as defined in the first aspect.

In a third aspect, the invention provides the use of one or more genes selected from the group consisting of NRP2, GSDMD, LOC102800447, NLRC5, GFRA1, LTBP3, and SPTLC; or alternatively the CpG position cg05387464; or alternatively one or more genes selected from the group consisting of NRP2, GSDMD, LOC102800447, NLRC5, GFRA1, LTBP3, and SPTLC, in combination with the CpG position cg05387464;as marker(s) for predicting the responsiveness of a subject to a weight loss intervention in an isolated test sample.

In a fourth aspect the invention provides the use of one or more CpG positions selected from the group consisting of cg14157435, cg00036352, cg05387464, cg08240913, cg18872420, cg16007266, cg23743554, and cg04057818 as marker(s) for predicting the responsiveness of a subject to a weight loss intervention in an isolated test sample.

In a fifth aspect the invention provides the use of a kit for predicting the responsiveness of a subject to a weight loss intervention, the kit comprising a solid support and means for determining the methylation status of one or more genes selected from the group consisting of NRP2, GSDMD, LOC102800447, NLRC5, GFRA1, LTBP3, and SPTLC2; or alternatively CpG position cg05387464; or alternatively one or more genes selected from the group consisting of NRP2, GSDMD, LOC102800447, NLRC5, GFRA1, LTBP3, and SPTLC2, in combination with the CpG position cg05387464.

## Detailed description of the invention

All terms as used herein in this application, unless otherwise stated, shall be understood in their ordinary meaning as known in the state-of-the-art. Other more specific definitions for certain terms as used in the present application are as set forth below and are intended to apply uniformly through-out the specification and claims unless an otherwise expressly set out definition provides a broader definition.

As mentioned above, in a first aspect the present invention provides a methylation status-based method for predicting the responsiveness of a subject to a non-surgical weight loss intervention.

As used herein, "weight loss intervention" refers to any non-surgical intervention in a subject aimed at reducing his/her body weight, such as an adjustment of the diet, physical exercise, the administration of weight loss drugs, or combinations thereof. As used herein, the term "diet" refers to any feeding regime that restricts the total calories consumed by the subject to lower his/her weight maintenance level, regardless of any preference for a macronutrient. "Physical exercise" refers to any plan of physical activity designed with the purpose of increasing the total daily energy expenditure of the subject. In one embodiment, optionally in combination with any embodiment provided above or below, the weight loss intervention comprises diet or physical exercise. In another embodiment, the weight loss intervention comprises diet and physical exercise. In another embodiment, the weight loss intervention comprises a low caloric diet and moderate exercise. "Low caloric diet" refers to a diet that provides to the subject about 50% of the calories of a standard diet. The skilled in the art would know which is the standard diet of a subject depending of his/her age and gender. "Moderate exercise" refers to 30 minutes of aerobic physical exercise per day.

The term "methylation status of a gene" as used herein refers to the DNA methylation status of a gene, i.e. the presence or absence of a methyl group in one or more nucleotides located within the gene sequence, including its promoter and the rests of its elements. In one embodiment, the methylation status of a gene is determined in one or more CpG positions. A "CpG position" is a DNA region where a cytosine nucleotide is followed by a guanine nucleotide in the linear sequence of bases along its 5' → 3' direction. Cytosines in CpG dinucleotides can be methylated to form 5-methylcytosine.

"NRP2" refers to the human gene also known as neuropilin 2, P2, NPN2, PRO2714, and VEGF165R2. The gene sequence is available in several genetic sequence databases, such as in GeneBank (Gene ID: 8828, updated on 19-Aug-2018).

"NLRC5" refers to the human gene also known as NLR family CARD domain containing 5, CLR16.1, NOD27, and NOD4. The gene sequence is available in several genetic sequence databases, such as in GeneBank (Gene ID: 84166, updated on 14-Oct-2018).

"GSDMD" refers to the human gene also known as gasdermin D, DF5L, DFNA5L, FKSG10C1, and GSDMD. The gene sequence is available in several genetic sequence databases, such as in GeneBank (Gene ID: 79792, updated on 9-Sep-2018).

"LOC102800447" refers to the human gene also known as long intergenic non-protein coding RNA 1828 and LINC01828. The gene sequence is available in several genetic sequence databases, such as in GeneBank (Gene ID: 102800447, updated on 29-Mar-2018).

"GFRA1" refers to the human gene also known as GDNF family receptor alpha 1, GDNFR, GDNFRA, GFR-ALPHA-1, RET1L, RETL1, and TRNR1. The gene sequence is available in several genetic sequence databases, such as in GeneBank (Gene ID: 2674, updated on 12-Aug-2018).

"LTBP3" refers to the human gene also known as latent transforming growth factor beta binding protein 3, DASS, GPHYSD3, LTBP-3, LTBP2, STHAG6, and pp6425. The gene sequence is available in several genetic sequence databases, such as in GeneBank (Gene ID: 4054, updated on 15-Oct-2018).

"SPTLC2" refers to the human gene also known as serine palmitoyltransferase long chain base subunit 2, HSN1C, LCB2, LCB2A, NSAN1C, SPT2, and hLCB2a. The gene sequence is available in several genetic sequence databases, such as in GeneBank (Gene ID: 9517, updated on 5-Aug-2018).

In a particular embodiment of the first aspect, optionally in combination with any embodiment provided above or below, the subject suffers from overweight or obesity.

In a particular embodiment of the first aspect, optionally in combination with any embodiment provided above or below, the subject is a child. In another particular embodiment of the first aspect of the invention, optionally in combination with any of the embodiments provided above or below, the subject is an obese child. As used herein, the term "child" refers to a subject from 3 to 12 years of age, more particularly from 6 to 12 years of age, and even more particularly from 7 to 10 years of age.

In a particular embodiment of the first aspect, optionally in combination with any embodiment provided above or below, the methylation status is determined in at least two of the markers. In a particular embodiment, the methylation status is determined in two markers. In a particular embodiment, the methylation status is determined in one or more sets of markers selected from: NRP2, GSDMD; NRP2, LOC102800447; NRP2, NLRC5; NRP2, GFRA1; NRP2, LTBP3; NRP2, SPTLC2; NRP2, cg05387464; GSDMD, LOC102800447; GSDMD, NLRC5; GSDMD, GFRA1; GSDMD, LTBP3; GSDMD, SPTLC2; GSDMD, cg05387464; LOC102800447, NLRC5; LOC102800447, GFRA1; LOC102800447, LTBP3; LOC102800447, SPTLC2; LOC102800447, cg05387464; NLRC5, GFRA1; NLRC5,LTBP3; NLRC5, SPTLC2; NLRC5, cg05387464; GFRA1,LTBP3; GFRA1, SPTLC2; GFRA1, cg05387464; LTBP3, SPTLC2; LTBP3, cg05387464; SPTLC2, cg05387464. In a more particular embodiment, the methylation status is determined in at least NRP2 and NLRC5. In a more particular embodiment, the methylation status is only determined in NRP2 and NLRC5. As it is shown below, the predictive power of the method was further strengthened when two markers were considered (see Table 6).

In a particular embodiment of the first aspect, optionally in combination with any embodiment provided above or below, the methylation status is determined in at least four markers. In a particular embodiment, the methylation status is determined in four markers. In another embodiment of the first aspect of the invention, it is determined the methylation status of one or more sets selected from: NRP2,GSDMD,LOC102800447,NLRC5; NRP2,GSDMD,LOC102800447,GFRA1; NRP2,GSDMD,LOC102800447,LTBP3; NRP2,GSDMD,LOC102800447,SPTLC2; NRP2,GSDMD,LOC102800447,cg05387464; NRP2,GSDMD,NLRC5,GFRA1; NRP2,GSDMD,NLRC5,LTBP3; NRP2,GSDMD,NLRC5,SPTLC2; NRP2,GSDMD,NLRC5,cg05387464; NRP2,GSDMD,GFRA1,LTBP3; NRP2,GSDMD,GFRA1,SPTLC2; NRP2,GSDMD,GFRA1,cg05387464; NRP2,GSDMD,LTBP3,SPTLC2; NRP2,GSDMD,LTBP3,cg05387464; NRP2,GSDMD,SPTLC2,cg05387464; NRP2,LOC102800447,NLRC5,GFRA1; NRP2,LOC102800447,NLRC5,LTBP3; NRP2,LOC102800447,NLRC5,SPTLC2; NRP2,LOC102800447,NLRC5,cg05387464; NRP2,LOC102800447,GFRA1,LTBP3; NRP2,LOC102800447,GFRA1,SPTLC2; NRP2,LOC102800447,GFRA1,cg05387464; NRP2,LOC102800447,LTBP3,SPTLC2; NRP2,LOC102800447,LTBP3,cg05387464; NRP2,LOC102800447,SPTLC2,cg05387464; NRP2,NLRC5,GFRA1,LTBP3; NRP2,NLRC5,GFRA1,SPTLC2; NRP2,NLRC5,GFRA1,cg05387464; NRP2,NLRC5,LTBP3,SPTLC2; NRP2,NLRC5,LTBP3,cg05387464; NRP2,NLRC5,SPTLC2,cg05387464; NRP2,GFRA1,LTBP3,SPTLC2; NRP2,GFRA1,LTBP3,cg05387464; NRP2,GFRA1,SPTLC2,cg05387464; NRP2,LTBP3,SPTLC2,cg05387464; GSDMD,LOC102800447,NLRC5,GFRA1; GSDMD,LOC102800447,NLRC5,LTBP3; GSDMD,LOC102800447,NLRC5,SPTLC2; GSDMD,LOC102800447,NLRC5,cg05387464; GSDMD,LOC102800447,GFRA1,LTBP3; GSDMD,LOC102800447,GFRA1,SPTLC2; GSDMD,LOC102800447,GFRA1,cg05387464; GSDMD,LOC102800447,LTBP3,SPTLC2; GSDMD,LOC102800447,LTBP3,cg05387464; GSDMD,LOC102800447,SPTLC2,cg05387464; GSDMD,NLRC5,GFRA1,LTBP3; GSDMD,NLRC5,GFRA1,SPTLC2; GSDMD,NLRC5,GFRA1 ,cg05387464; GSDMD,NLRC5,LTBP3,SPTLC2; GSDMD,NLRC5,LTBP3,cg05387464; GSDMD,NLRC5,SPTLC2,cg05387464; GSDMD,GFRA1,LTBP3,SPTLC2; GSDMD,GFRA1,LTBP3,cg05387464; GSDMD,GFRA1,SPTLC2,cg05387464; GSDMD,LTBP3,SPTLC2,cg05387464; LOC102800447,NLRC5,GFRA1,LTBP3; LOC102800447,NLRC5,GFRA1,SPTLC2; LOC102800447,NLRC5,GFRA1,cg05387464; LOC102800447,NLRC5,LTBP3,SPTLC2; LOC102800447,NLRC5,LTBP3,cg05387464; LOC102800447,NLRC5,SPTLC2,cg05387464; LOC102800447,GFRA1,LTBP3,SPTLC2; LOC102800447,GFRA1,LTBP3,cg05387464; LOC102800447,GFRA1,SPTLC2,cg05387464; LOC102800447,LTBP3,SPTLC2,cg05387464; NLRC5,GFRA1,LTBP3,SPTLC2; NLRC5,GFRA1,LTBP3,cg05387464; NLRC5,GFRA1,SPTLC2,cg05387464; NLRC5,LTBP3,SPTLC2,cg05387464; GFRA1,LTBP3,SPTLC2,cg05387464. In a more particular embodiment, the methylation status is determined in at least NRP2, NLRC5, GSDMD, and LOC102800447. In a more particular embodiment, methylation status is determined in NRP2, NLRC5, GSDMD, and LOC102800447. Importantly, the inventors also found that combining the information provided by the methylation of the four markers NRP2, NLRC5, LOC102800447, and GSDMD, the predictive power of the method was further strengthened (see Table 7).

In a particular embodiment of the first aspect, optionally in combination with any embodiment provided above or below, the methylation status is determined in the genes NRP2, NLRC5, GSDMD, LOC102800447, GFRA1, LTBP3, and SPTLC2.

In a particular embodiment of the first aspect, optionally in combination with any embodiment provided above or below, the method comprises the step of determining the methylation status of the genes NRP2, NLRC5, GSDMD, LOC102800447, GFRA1, LTBP3, SPTLC2, and the CpG position cg05387464. The inventors also found that the information provided by the combination of these eight markers, improved the predictive power of the method achieving outstanding values of sensitivity and specificity.

In another embodiment of the method of the first aspect of the invention, it is determined the methylation status in one or more CpG positions of one or more of the gene markers. In another embodiment of the method of the first aspect of the invention, it is determined the methylation status of one CpG position in one or more gene markers. In another embodiment of the method of the first aspect of the invention, it is determined the methylation status in one or more CpG positions in two of the gene markers. In another embodiment of the method of the first aspect of the invention, it is determined the methylation status of one CpG position in two gene markers.

In a particular embodiment of the first aspect, optionally in combination with any embodiment provided above or below, the determining of the methylation status of the gene comprises determining is the methylation status of one or more CpG positions located within the gene.

In a particular embodiment of the first aspect, optionally in combination with any embodiment provided above or below, the one or more CpG positions are selected from the group consisting of:

- the CpG position cg14157435 of the NRP2 gene;
- the CpG position cg16007266 of the NLRC5 gene;
- the CpG position cg00036352 of the GSDMD gene;
- the CpG position cg04057818 of the LOC102800447 gene;
- the CpG position cg08240913 of the GFRA1 gene;
- the CpG position cg23743554 of the LTBP3 gene;
- the CpG position cg18872420 of the SPTLC2 gene,

and combinations thereof.

In another embodiment of the first aspect, optionally in combination with any of the embodiments provided above or below, it is determined the methylation status of CpG position cg14157435 in the NRP2 gene.

In another embodiment of the first aspect, optionally in combination with any of the embodiments provided above or below, it is determined the methylation status of CpG position cg16007266 in the NLRC5 gene.

In another embodiment of the first aspect, optionally in combination with any of the embodiments provided above or below, it is determined the methylation status of CpG position cg00036352 in the GSDMD gene.

In another embodiment of the first aspect, optionally in combination with any of the embodiments provided above or below, it is determined the methylation status of CpG position cg04057818 in the LOC102800447 gene.

In another embodiment of the first aspect, optionally in combination with any of the embodiments provided above or below, it is determined the methylation status of CpG position cg08240913 in the GFRA1 gene.

In another embodiment of the first aspect, optionally in combination with any of the embodiments provided above or below, it is determined the methylation status of CpG position cg23743554 in the LTBP3 gene.

In another embodiment of the first aspect, optionally in combination with any of the embodiments provided above or below, it is determined the methylation status of CpG position cg18872420 in the SPTLC2 gene.

In another embodiment of the method of the first aspect of the invention, optionally in combination with any embodiment provided above or below, it is determined the methylation status of one or more of the following CpG sets:

cg14157435,cg00036352;     cg14157435,cg05387464;     cg14157435,cg08240913;     cg14157435,cg18872420;
cg14157435,cg16007266;     cg14157435,cg23743554;     cg14157435,cg04057818;     cg00036352,cg05387464;
cg00036352,cg08240913;     cg00036352,cg18872420;     cg00036352,cg16007266;     cg00036352,cg23743554;
cg00036352,cg04057818;     cg05387464,cg08240913;     cg05387464,cg18872420;     cg05387464,cg16007266;
cg05387464,cg23743554;     cg05387464,cg04057818;     cg08240913,cg18872420;     cg08240913,cg16007266;
cg08240913,cg23743554;     cg08240913,cg04057818;     cg18872420,cg16007266;     cg18872420,cg23743554;
cg18872420,cg04057818; cg16007266,cg23743554; cg16007266,cg04057818; cg23743554,cg04057818.

Alternatively, in another embodiment of the method of the first aspect of the invention, optionally in combination with any embodiment provided above or below, it is determined the methylation status of one or more of the following CpG sets:

cg14157435,cg00036352,cg05387 464,cg08240913;
cg14157435,cg00036352,cg05387 464,cg18872420;
cg14157435,cg00036352,cg05387 464,cg16007266;
cg14157435,cg00036352,cg05387 464,cg237 43554;

cg14157435,cg00036352,cg05387464,cg04057818;
cg14157435,cg00036352,cg08240913,cg18872420;
cg14157435,cg00036352,cg08240913,cg16007266;
cg14157435,cg00036352,cg08240913,cg237 43554;
cg14157435,cg00036352,cg08240913,cg04057818;
cg14157435,cg00036352,cg18872420,cg16007266;
cg14157435,cg00036352,cg18872420,cg237 43554;
cg14157435,cg00036352,cg18872420,cg04057818;
cg14157435,cg00036352,cg16007266,cg237 43554;
cg14157435,cg00036352,cg16007266,cg04057818;
cg14157435,cg00036352,cg23743554,cg04057818;
cg14157435,cg05387464,cg08240913,cg18872420;
cg14157435,cg05387464,cg08240913,cg16007266;
cg14157435,cg05387464,cg08240913,cg237 43554;
cg14157435,cg05387464,cg08240913,cg04057818;
cg14157435,cg05387464,cg18872420,cg16007266;
cg14157435,cg05387464,cg18872420,cg23743554;
cg14157435,cg05387464,cg18872420,cg04057818;
cg14157435,cg05387464,cg16007266,cg23743554;
cg14157435,cg05387464,cg16007266,cg04057818;
cg14157435,cg05387464,cg23743554,cg04057818;
cg14157435,cg08240913,cg18872420,cg16007266;
cg14157435,cg08240913,cg18872420,cg237 43554;
cg14157435,cg08240913,cg18872420,cg04057818;
cg14157435,cg08240913,cg16007266,cg23743554;
cg14157435,cg08240913,cg16007266,cg04057818;
cg14157435,cg08240913,cg23743554,cg04057818;
cg14157435,cg18872420,cg16007266,cg23743554;
cg14157435,cg18872420,cg16007266,cg04057818;
cg14157435,cg18872420,cg23743554,cg04057818;
cg14157435,cg16007266,cg23743554,cg04057818;
cg00036352,cg05387464,cg08240913,cg18872420;
cg00036352,cg05387464,cg08240913,cg16007266;
cg00036352,cg05387464,cg08240913,cg23743554;
cg00036352,cg05387464,cg08240913,cg04057818;
cg00036352,cg05387464,cg18872420,cg16007266;
cg00036352,cg05387464,cg18872420,cg23743554;
cg00036352,cg05387464,cg18872420,cg04057818;
cg00036352,cg05387464,cg16007266,cg23743554;
cg00036352,cg05387464,cg16007266,cg04057818;
cg00036352,cg05387464,cg23743554,cg04057818;
cg00036352,cg08240913,cg18872420,cg16007266;
cg00036352,cg08240913,cg18872420,cg23743554;
cg00036352,cg08240913,cg18872420,cg04057818;
cg00036352,cg08240913,cg16007266,cg23743554;
cg00036352,cg08240913,cg16007266,cg04057818;
cg00036352,cg08240913,cg23743554,cg04057818;
cg00036352,cg18872420,cg16007266,cg23743554;
cg00036352,cg18872420,cg16007266,cg04057818;
cg00036352,cg18872420,cg23743554,cg04057818;
cg00036352,cg16007266,cg23743554,cg04057818;
cg05387464,cg08240913,cg18872420,cg16007266;
cg05387464,cg08240913,cg18872420,cg23743554;
cg05387464,cg08240913,cg18872420,cg04057818;
cg05387464,cg08240913,cg16007266,cg23743554;
cg05387464,cg08240913,cg16007266,cg04057818;
cg05387464,cg08240913,cg23743554,cg04057818;
cg05387464,cg18872420,cg16007266,cg23743554;

cg05387464,cg18872420,cg16007266,cg04057818;
cg05387464,cg18872420,cg23743554,cg04057818;
cg05387464,cg16007266,cg23743554,cg04057818;
cg08240913,cg18872420,cg16007266,cg23743554;
cg08240913,cg18872420,cg16007266,cg04057818;
cg08240913,cg18872420,cg23743554,cg04057818;
cg08240913,cg16007266,cg23743554,cg04057818;
cg18872420,cg16007266,cg23743554,cg04057818.

In another embodiment of the first aspect, optionally in combination with any of the embodiments provided above or below, it is determined the methylation status of the CpG positions cg14157435, cg00036352, cg05387464, cg08240913, cg18872420, cg16007266, cg23743554, and cg04057818.

As used herein the term "high-responder" or "HR" refers to a subject that loses a significant amount of weight when subjected to a weight-loss intervention for an appropriate period of time, for example for at least 6 months. Consistently, the term "low-responder" or "LR" refers to a subject that does not lose a significant amount of weight when subjected to a weight loss intervention for an appropriate period of time, for example for at least 6 months.

The value commonly used to discriminate between HR and LR is the differential normalized BMI (DzBMI). As used herein, "DzBMI" is the difference of the zBMI before and after the weight loss intervention. "zBMI" refers to the BMI (body mass index) standard deviation score greater than two standard deviations for a given age and sex, following the World Health Organization (WHO) standards. Individual zBMI for a given age and sex was obtained by using "AnthroPlus" software (WHO).

Before the filing of the present patent application, the skilled person in the art already worked with DzBMI cutoff values previously known to be useful in discriminating HR from LR. Said cutoff values can also be used when performing the method of the invention. Thus, in one embodiment, the DzBMI cut-off value is -0.3, meaning that a HR would be any subject with a predicted DzBMI value lower or equal to -0.3, and a LR would be any subject with a predicted DzBMI value higher than -0.3. In another embodiment, it is used a double cut-off value: a HR would be any subject with a predicted DzBMI value lower or equal to -0.3 and a LR would be any subject with a predicted DzBMI value higher than - 0.1. Although using these well-established cut-off values in the method of the invention the skilled person has a reliable tool to classify subjects, the optimal sensitivity and specificity of the method might not be reached.

The present inventors following the predictive models provided below in the Examples section have found optimal DzBMI cut-off values for each predictive model. For instance, when the eight biomarkers are used, the optimal DzBMI cutoff value is -0.403. As it is illustrated below, using this optimal cut-off the subjects can be properly classified as HR or LR with a 100% specificity.

Thus, in another embodiment of the first aspect, optionally in combination with any of the embodiments provided above or below, the method further comprises the step of determining the predicted differential body mass index z-score (DzBMI),
wherein if the DzBMI is lower than or equal to -0.403, this is indicative that the individual will be a high-responder to the weight loss intervention; and
wherein if the DzBMI is higher than -0.403, this is indicative that the individual will be a low-responder to the weight loss intervention.

In another embodiment of the first aspect, optionally in combination with any of the embodiments provided above or below, the method further comprises the step of determining the predicted differential body mass index z-score (DzBMI) with the formula (I):

$$DzBMI = -0.744 + (-0.08m_1) + (-0.077m_2) + (-0.049m_3) + (-0.05m_4) + (-0.068m_5) + (-0.084m_6) + (-0.058m_7) + (0.082m_8) \quad (I)$$

wherein m1 is the log2 ratio of methylated probe intensity and unmethylated probe intensity for cg14157435;
wherein m2 is the log2 ratio of methylated probe intensity and unmethylated probe intensity for cg00036352;
wherein m3 is the log2 ratio of methylated probe intensity and unmethylated probe intensity for cg05387464;
wherein m4 is the log2 ratio of methylated probe intensity and unmethylated probe intensity for cg08240913;
wherein m5 is the log2 ratio of methylated probe intensity and unmethylated probe intensity for cg18872420;
wherein m6 is the log2 ratio of methylated probe intensity and unmethylated probe intensity for cg16007266;
wherein m7 is the log2 ratio of methylated probe intensity and unmethylated probe intensity for cg23743554;
wherein m8 is the log2 ratio of methylated probe intensity and unmethylated probe intensity for cg04057818;
wherein if the DzBMI is lower than or equal to -0.403, this is indicative that the individual will be a high-responder to the weight loss intervention; and

wherein if the DzBMI is higher than -0.403, this is indicative that the individual will be a low-responder to the weight loss intervention.

In a particular embodiment of the first aspect, optionally in combination with any embodiment provided above or below, the isolated test sample is selected from the group consisting of serum, plasma, saliva, pleural, cerebral spinal fluid (CSF), blood, amniotic fluid, urine, feces, mucus, cell extracts and pus. In a more particular embodiment, the isolated test sample is blood.

As mentioned above, the present invention also provides the use of means for determining the methylation status of the markers at hand for predicting the responsiveness of a subject to a weight loss intervention as defined in the first aspect.

The term "means" refers to the reactants or apparatus used for the determination of DNA methylation. For instance, for the detection of DNA methylation the reactants commonly used are methylation arrays (such as Illumina EPIC 850K arrays® kit or Illumina 450K arrays® kit), methylation sensitive primers, bisulfite (pyrosequencing), etc.

In a particular embodiment of the second aspect, optionally in combination with any embodiment provided above or below, the means are for determining in a biological sample the methylation status of:

- one or more genes selected from the group consisting of NRP2, GSDMD, LOC102800447, NLRC5, GFRA1, LTBP3, and SPTLC2; or alternatively

- the CpG position cg05387464; or alternatively

- one or more genes selected from the group consisting of NRP2, GSDMD, LOC102800447, NLRC5, GFRA1, LTBP3, and SPTLC2, in combination with CpG position cg05387464;

in a method for predicting the responsiveness of a subject to a weight loss intervention as defined in the first aspect, and wherein optionally, the methylation status of the one or more genes is the methylation status of a CpG position selected from the group consisting of:

- the CpG position cg14157435 of the NRP2 gene;
- the CpG position cg16007266 of the NLRC5 gene;
- the CpG position cg00036352 of the GSDMD gene;
- the CpG position cg04057818 of the LOC102800447 gene;
- the CpG position cg08240913 of the GFRA1 gene;
- the CpG position cg23743554 of the LTBP3 gene;
- the CpG position cg18872420 of the SPTLC2 gene; and
- combinations thereof.

All the embodiments provided under the first aspect of the invention, regarding the determination of two, four or all gene markers, as well as the different sets of markers provided above (both of gene markers as well as of CpG positions), and the weight loss intervention, are also embodiments of the second aspect of the invention.

In a more particular embodiment of the second aspect, the means are for determining the methylation status of a CpG position selected from the group consisting of cg14157435, cg00036352, cg05387464, cg08240913, cg18872420, cg16007266, cg23743554, cg04057818, and combinations thereof. In a more particular embodiment, the means are for determining the methylation status of the CpG positions cg14157435 and cg16007266. In a more particular embodiment, the means are for determining the methylation status of the CpG positions cg14157435, cg16007266, cg04057818 and cg00036352. In a more particular embodiment, the means are for determining the methylation status of the CpG positions cg14157435, cg00036352, cg05387464, cg08240913, cg18872420, cg16007266, cg23743554 and cg04057818.

In a particular embodiment of the second aspect, optionally in combination with any embodiment provided above or below, the means form part of a kit.

As mentioned above, the invention also provides the use of one or more genes selected from the group consisting of NRP2, GSDMD, LOC102800447, NLRC5, GFRA1, LTBP3, and SPTLC; or the CpG position cg05387464; as marker(s) in a method for predicting the responsiveness of a subject to a weight loss intervention as defined in in the first aspect of the invention.

All the embodiments provided under the first aspect of the invention, regarding the determination of two, four or all gene markers, as well as the different sets of markers provided above, and the weight loss intervention are also embodiments of the third aspect of the invention.

In a more particular embodiment of the third aspect, the invention also provides the use of one or more genes selected from the group consisting of NRP2, GSDMD, LOC102800447, NLRC5, GFRA1, LTBP3, and SPTLC, in combination with the CpG position cg05387464, as marker(s) in a method for predicting the responsiveness of a subject to a weight loss intervention as defined in in the first aspect of the invention.

In a more particular embodiment of the third aspect, the invention provides the use of the genes NRP2, GSDMD, LOC102800447, NLRC5, GFRA1, LTBP3, and SPTLC, in combination with the CpG position cg05387464, as marker(s) in a method for predicting the responsiveness of a subject to a weight loss intervention as defined in in the first aspect of the invention. In a particular embodiment, the invention provides the use of the genes NRP2 and NLRC5 as marker(s) in a method for predicting the responsiveness of a subject to a weight loss intervention as defined in in the first aspect of the invention. In a particular embodiment, the invention provides the use of the genes NRP2, NLRC5, GSDMD and LOC102800447 as marker(s) in a method for predicting the responsiveness of a subject to a weight loss intervention as defined in in the first aspect of the invention

As mentioned above, the invention also provides the use of one or more CpG positions selected from the group consisting of cg14157435, cg00036352, cg05387464, cg08240913, cg18872420, cg16007266, cg23743554, and cg04057818 as marker(s) for predicting the responsiveness of a subject to a weight loss intervention in an isolated test sample.

In a particular embodiment of the fourth aspect, optionally in combination with any embodiment provided above or below, the invention provides the use of at least two CpG positions selected from the group consisting of cg14157435, cg00036352, cg05387464, cg08240913, cg18872420, cg16007266, cg23743554, and cg04057818 for predicting the responsiveness of a subject to a weight loss intervention. In a particular embodiment, the invention provides the use of at least four CpG positions selected from the group consisting of cg14157435, cg00036352, cg05387464, cg08240913, cg18872420, cg16007266, cg23743554, and cg04057818 for predicting the responsiveness of a subject to a weight loss intervention. In a particular embodiment, the invention provides the use of the CpG positions cg14157435, cg00036352, cg05387464, cg08240913, cg18872420, cg16007266, cg23743554, and cg04057818 for predicting the responsiveness of a subject to a weight loss intervention. In a particular embodiment, the invention provides the use of the CpG positions cg14157435 and cg16007266 for predicting the responsiveness of a subject to a weight loss intervention. In a particular embodiment, the invention provides the use of the CpG positions cg14157435, cg16007266, cg04057818 and cg00036352 for predicting the responsiveness of a subject to a weight loss intervention.

As mentioned before, the invention also provides the use of a kit for predicting the responsiveness of a subject to a weight loss intervention.

In a particular embodiment of the fifth aspect, optionally in combination with any embodiment provided above or below, the means are for determining the methylation status of:

- one or more genes selected from the group consisting of NRP2, GSDMD, LOC102800447, NLRC5, GFRA1, LTBP3, and SPTLC2; or alternatively

- the CpG position cg05387464; or alternatively

- one or more genes selected from the group consisting of NRP2, GSDMD, LOC102800447, NLRC5, GFRA1, LTBP3, and SPTLC2, in combination with the CpG position cg05387464.

In a particular embodiment of the fifth aspect, optionally in combination with any embodiment provided above or below, the means are for determining the methylation status of a CpG position is selected from the group consisting of:

- the CpG position cg14157435 of the NRP2 gene;
- the CpG position cg16007266 of the NLRC5 gene;
- the CpG position cg00036352 of the GSDMD gene;
- the CpG position cg04057818 of the LOC102800447 gene;
- the CpG position cg08240913 of the GFRA1 gene;
- the CpG position cg23743554 of the LTBP3 gene;
- the CpG position cg18872420 of the SPTLC2 gene,

and combinations thereof.

All the embodiments of the first aspect related to the subject, the weight loss intervention or the combinations of genes and CpG positions are also meant to apply to the fifth aspects of the invention.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

**Examples**

**Biomarkers for the prediction of weight loss intervention response in children with severe obesity**

1. Experimental design

1.1. Subjects included in the study

From the research group of metabolic endocrinology of the Research Institute of the Pediatric Hospital Sant Joan de Déu, a clinical cohort of 26 pre-pubertal children (17 boys and 9 girls) with severe obesity were analyzed. The premature appearance of girls' puberty with respect to boys was considered by only selecting those subjects who strictly complied with the conditions of the pre-pubertal phase. Individuals were treated by entering in a nutritional and exercise program to reduce weight which consisted of a period of 6 months under a diet and moderate exercise as described below.
Personalized counseling was achieved with an individual interview. Motivational interviewing were used primarily focused on behavioral changes to improve lifestyle of all family members. Nutritional counseling followed the recommendations of the Department of Health of the autonomous government of Catalonia (Spain), based on the Mediterranean diet and in agreement with the WHO. Such diet consists in 55% of kcal from carbohydrates (less than 10% of sugars), 15% of kcal from protein, and 30% of kcal from lipids (less than 10% saturated fat). Visual laminated support material was used, including food models and plates, to educate on portion size. Help was provided to patients and families designing food menus emphasizing the importance of variety and quality, as well as the cooking method. Additionally, participants were encouraged to choose healthy nutrition options and to incorporate a minimum of 30 min of physical exercise per day into their lives. Subjects had follow-up interviews with the nutritionist 2 weeks and 3 months after the initial interview, to review changes made and set new goals (Leal-Witt MJ et al., "Lifestyle Intervention Decreases Urine Trimethylamine N-Oxide Levels in Prepubertal Children with Obesity", Obesity (Silver Spring), 2018, vol. 26(10), pp. 1603-1610).

1.2. Cohort characterization

The individual age as the one at the time of initiating the treatment was considered. The weight and height of each subject was measured before starting the treatment and after its 6 months of duration. From these values, their Body Mass Index (BMI) was calculated as indicated in the equation:

$$BMI = \frac{Weight\,[kg]}{Height^2\,[m]}$$

To obtain a meaningful BMI in children, the BMI z-score (zBMI) was computed before and after treatment from previous BMI scores. This measure consists of the BMI value corrected for child age and sex.
The difference of zBMI (DzBMI) before and after the program was not homogeneous across subjects by identifying a group that responded better to treatment and another group that responded worse in terms of weight reduction. Based on this, individuals who responded well to treatment were qualified as high responders (HR) and those who responded poorly as low responders (LR).
The criterion for discriminating the two groups consisted of a DzBMI value of -0.3. Smaller values indicated that the subject was HR and higher or equal values to -0.1 indicated that the individual was LR. Values between -0.3 and -0.1 were also indicative of LR. On that basis, 13 HR (9 boys and 4 girls) and 13 LR (8 boys and 5 girls) were obtained. Table 1 indicates the individual distribution across the two groups as well as the mean age and corresponding standard deviation in years.

**Table 1**

|  | HR | LR |
|---|---|---|
| Boys | 9(8.8±1.3) | 8(9.3±1.3) |

(continued)

| | HR | LR |
|---|---|---|
| Girls | 4(8.5 ±0.77) | 5(8.6 ±0.88) |
| TOTAL | 13(8.7 ±1.1) | 13(9.0 ±1.2) |

Distribution of subjects per gender. In brackets, the mean age and corresponding standard deviation

1.3. DNA Methylation profiling of CpG islands

To characterize the global pattern of DNA methylation of CpG islands, blood samples were extracted from the already stratified children before starting the treatment following standard procedures. Illumina EPIC 850K arrays® methylation array was used to estimate the methylation level. For this purpose, two different metrics were obtained:

- Beta-value: Ratio between Illumina methylated probe intensity and total probe intensities (sum of methylated and unmethylated probe intensities). It is in the range of 0 and 1, which can be interpreted as the percentage of methylation. However, the Beta-value metric has severe heteroscedasticity in the low and high methylation range, which imposes serious challenges in applying many statistic models.

- M-value: Log2 ratio of methylated probe intensity and unmethylated probe intensity is another method used to measure the methylation level. The M-value metric is approximately homoscedastic in the entire methylation range. As a result, it is more statistically valid in differential and other statistical analysis.

The relationship between Beta-Value and M-value is given in the equation:

$$Beta_i = \frac{2^{M_i}}{1 + 2^{M_i}}; \; M_i = log_2 \left( \frac{Beta_i}{1 - Beta_i} \right)$$

1.4. Filtering (Quality control)

ChAMP R package was used to filter the array probes (Morris T.J. et al., "ChAMP: 450k Chip Analysis Methylation Pipeline", Bioinformatics, 2014, vol. 30(3), pp. 428-30). The probes were discarded according to the following criteria (ChAMP default criteria):

• Probes with a detection p-value above 0.01 in one or more samples.
• Probes with a beadcount <3 in at least 5% of samples.
• Probes associated to SNPs.
• Probes that align to multiple locations.
• Probes located on the X or Y chromosome.

A total of 788,373 probes interrogating CpG sites were available after this filtering.

1.5. Normalization

After the filtering process, intra-array normalization was performed to adjust the data for bias introduced by the Infinium type 2 probe design used in the selected array platform. For this purpose, the Beta-Mixture Quantile (BMIQ) normalization method was used as the default approach implemented in ChAMP package.

1.6. Differential methylated CpG sites between HR and LR

To obtain the differentially methylated CpG sites between the HR and LR, the limma R package was used. For this purpose, a linear model (LM) was fit to the methylation levels of each interrogated CpG. To perform this LM, the M-values were used due to their homocedasticity property. The DzBMI values and sex of every individual were considered as model regressors as indicated in the following equation:

$$M - value\ (CpG_i) \sim \beta_0 + \beta_1 \times DzBMI + \beta_2 \times gender$$

The empirical Bayes moderated t-statistics tested each individual coefficient $\beta_i$ equal to zero within the limma package. Statistically significant differentially methylated CpG sites (differential CpGs) were selected and ranked for the DzBMI variable on the LM (adjusted p-value < 5%, FDR using the Benjamini-Hochberg procedure). To reduce the list of differential CpGs, those which showed an effect size <10% when comparing the beta-value average from the two groups of HR and LR were removed.

1.7. External Leave-One-Out during the differential methylated analysis

To minimize the overfitting effect of the small sample size in the construction of the prediction model, a set of iterations in the calculation of the differential CpGs that we called external Leave-One-Out (eLOO) was used. The eLOO process consisted on identifying as many lists of differential CpGs as samples available following the procedure from previous section. To obtain each list of differential CpGs sites, one different sample was excluded out of the N samples each time (N=26 in this case). In this way, the methylation levels of 25 samples (N-1) was only considered to determine every list and repeated the process N times.
At the end of the eLOO, there were N lists of differential CpGs, each one with a different number of statistically differential CpGs between the two phenotypes HR and LR.

1.8. Predictive model construction with the consensus CpGs

Among the N lists, those differential CpGs in common that were named as consensus CpGs were selected. To construct the predictive model, the M-values from the consensus CpGs and the corresponding DzBMI values from the individuals as indicated in the following equation were used:

$$DzBMI = w_0 + \sum_{i=1}^{i=k} w_i \times CpG_i$$

where DzBMI is a quantitative variable, $w_0$ is the PLSR model intercept, $w_i$ are the unknown weights for the corresponding M-value for each CpG in the consensus list and k is the number of CpGs that are included in that list. Each individual regression coefficient was tested with a t-statistics and null hypothesis equal to zero.
To fit this model, a Partial Least Squares Regression (PLSR) was used. Leave-one-out (LOO) cross validation was used for model assessment. To determine the optimum number of latent variables or components of the PLSR, the Root Mean Squared Error of Prediction (RMSEP) was inspected which is derived from the Predicted Residual Sum of Squares (PRESS), the latter being results from the LOO validation.

2. Results

Through the present method, the inventors found 8 consensus CpGs differentially methylated between HR and LR from an initial set of 788,373 CpG sites available after filtering (Table 2).

**Table 2**

|   | CpGID | Chrom | MAPINFO | UCSC RefGene Name | UCSC RefGene Group |
|---|-------|-------|---------|-------------------|--------------------|
| 1 | cg04057818 | 2 | 67487963 | LOC102800447 | Body |
| 2 | cg14157435 | 2 | 206628692 | NRP2 | Body |
| 3 | cg05387464 | 2 | 9956256 | - | Intergenic |
| 4 | cg00036352 | 8 | 144636448 | GSDMD | 5'UTR |
| 5 | cg16007266 | 16 | 57050314 | NLRC5 | TSS1500 |
| 6 | cg08240913 | 10 | 117969024 | GFRA1 | Body |
| 7 | cg23743554 | 11 | 65321226 | LTBP3 | Body |
| 8 | cg18872420 | 14 | 78023429 | SPTLC2 | Body |

List of consensus CpGs showing genome coordinates and gene, if any, located at that position.
The minimum RMSEP with 1 component was obtained as a result of building the PLSR model from the methylation levels of the consensus CpGs. Table 3 reflects the weights, according to the equation:

$$DzBMI = w_0 + \sum_{i=1}^{i=k} w_i \times CpG_i$$

for every CpG in the predictive model and considering 1 component.

**Table 3**

| CpG Id | Weight value |
|--------|--------------|
| cg14157435 | -0.080*** |
| cg00036352 | -0.077*** |
| cg05387464 | -0.049*** |
| cg08240913 | -0.050*** |
| cg18872420 | -0.068** |
| cg16007266 | -0.084*** |
| cg23743554 | -0.058*** |
| cg04057818 | 0.082*** |

Weight values in the PLSR prediciton model for every consensus CpG. Weight value for intercept is -0.744. A p-value is indicated for each weight value coded as *** < 0.001 and ** < 0.01.
The predicted against the observed DzBMI values obtained from the predictive model presented a Pearson's correlation coefficient of 0.85.
The predicted DzBMI values were used to classify the individuals into HR and LR. From this classification we obtained the ROC curve which achieves the maximum AUC with a DzBMI cutoff of -0.403 (AUC= 0.953, Sensitivity= 0.923, Specificity= 1).
Based on the DzBMI cutoff for the predicted values, Table 4 shows the confusion matrix with the real and predicted HR and LR individuals.

**Table 4**

| | HR Predicted | LR Predicted | TOTAL |
|--------|--------------|--------------|-------|
| HR Real | 13 | 0 | 13 |
| LR Real | 1 | 12 | 13 |
| TOTAL | 14 | 12 | 26 |

Confusion Matrix of the HR and LR classification of the predictive model based on the methylation level of the 8 consensus CpGs.
Importantly, the inventors also found that when any of these 8 CpG positions were used individually in a predictive model, they all provided strong statistical power to classify the individuals into HR and LR before treatment:

**Table 5**

| CpGID | AUC | Sensitivity | Specificity | Cutoff |
|-------|-----|-------------|-------------|--------|
| cg14157435 | 0.941 | 0.846 | 1 | -0.356 |
| cg04057818 | 0.964 | 1 | 0.846 | -0.648 |
| cg05387464 | 0.923 | 0.923 | 0.846 | -0.499 |
| cg00036352 | 0.905 | 0.923 | 0.769 | -0.508 |
| cg16007266 | 0.870 | 0.769 | 0.923 | -0.417 |
| cg08240913 | 0.899 | 1 | 0.769 | -0.587 |
| cg23743554 | 0.905 | 0.846 | 0.846 | -0.500 |
| cg18872420 | 0.911 | 0.923 | 0.846 | -0.525 |

Moreover, when the CpGs were used in different combinations the following values for the predictive models were obtained:

**Table 6**

| CpGIDs | AUC | Sensitivity | Specificity | Cutoff |
|---|---|---|---|---|
| cg14157435 and cg16007266 | 0.923 | 0.923 | 0.923 | -0.434 |

**Table 7**

| CpGIDs | AUC | Sensitivity | Specificity | Cutoff |
|---|---|---|---|---|
| cg14157435, cg16007266, cg00036352 and cg04057818 | 0.953 | 0.923 | 1 | -0.460 |

In summary, from the above data it can be concluded that the eight methylation markers found by the inventors are highly reliable for the predicting weight loss treatment response when used either individually or in combination. Therefore, the markers of the invention constitute a powerful tool to stratify patients into HR or LR before weight loss interventions.

3. Protocol for the use of the identified epigenetic biomarkers in patients

The first step of the process when the obese patient arrives at the hospital is to extract a sample of peripheral blood following standard procedures.

After the processing of the sample (the process of extracting the DNA without modifying or altering the epigenetic profile following standard procedures known in the art), the methylation level of the 8 CpG constituting the model is evaluated. This analysis can be done in two different ways:

a) by Illumina EPIC 850K arrays® kit, or alternative equivalent array systems If this kit is used we can directly use the M value provided by the Illumina kits. If an Illumina kit is used to evaluate methylation levels, it is necessary to normalize and filter the data previously in the same way as described in the previous sections; or, alternatively,

b) by pyrosequencing. This method directed specifically at the 8 CpG of interest is cheaper, faster, but less sensitive than the previous one (Busato F. et al., "Quantitative DNA Methylation Analysis at Single-Nucleotide Resolution by Pyrosequencing®", Methods Mol Biol., 2018, vol. 1708, pp. 427-445); or alternatively

c) by bisulfite-sequencing (through either Sanger sequencing or NGS approaches).

Once obtained the M values (or its approximation) for each of the 8 CpG would be replaced in the following model: Or in the formula:

$$DzBMI \sim w_0 + \sum_{i=1}^{i=k} w_i \cdot CpG_i$$

DzBMI=-0.744+(-0.08m1)+(-0.077m2)+(-0.049m3)+(-0.05m4)+(-0.068m5)+(-0.084m6) +(-0.058m7)+(0.082m8) [where m1 is the log2 ratio of methylated probe intensity and unmethylated probe intensity for cg14157435; where m2 is the log2 ratio of methylated probe intensity and unmethylated probe intensity for cg00036352; where m3 is the log2 ratio of methylated probe intensity and unmethylated probe intensity for cg05387464; where m4 is the log2 ratio of methylated probe intensity and unmethylated probe intensity for cg08240913; where m5 is the log2 ratio of methylated probe intensity and unmethylated probe intensity for cg18872420; where m6 is the log2 ratio of methylated probe intensity and unmethylated probe intensity for cg16007266; where m7 is the log2 ratio of methylated probe intensity and unmethylated probe intensity for cg23743554; where m8 is the log2 ratio of methylated probe intensity and unmethylated probe intensity for cg04057818.]

If this DzMBI estimated value is greater than -0.403 the individual will be classified as "Low Responder" (LR), determining that this individual will not respond optimally to treatment and therefore, an alternative solution to the established treatment will be sought.

Otherwise, if the value of the estimate is less than -0.403, the individual will be qualified as "High Responder" (HR), determining that the individual will respond correctly to the treatment. In this case, the treatment established to treat the subject will proceed.

**Citation List**

Milagro F.I. et al., "A dual epigenomic approach for the search of obesity biomarkers: DNA methylation in relation to diet-induced weight loss" FASEB J. 2011; vol. 25(4), pp. 1378-89.

Xu C.J. et al., "The emerging landscape of dynamic DNA methylation in early childhood", BMC Genomics., 2017, vol. 18(1), pp. 25.

J. Tyson DeAngelis et al., "An Overview of Epigenetic Assays", 2008, Mol Biotechnol, vol. 38(2), pp. 179-183.

Burtis C. A. et al., 2008, Chapter 14, section "Statistical Treatment of Reference Values".

Leal-Witt MJ et al., "Lifestyle Intervention Decreases Urine Trimethylamine N-Oxide Levels in Prepubertal Children with Obesity", Obesity (Silver Spring), 2018, vol. 26(10), pp. 1603-1610.

Morris T.J. et al., "ChAMP: 450k Chip Analysis Methylation Pipeline", Bioinformatics, 2014, vol. 30(3), pp. 428-30.

Busato F. et al., "Quantitative DNA Methylation Analysis at Single-Nucleotide Resolution by Pyrosequencing®", Methods Mol Biol., 2018, vol. 1708, pp. 427-445.

**Claims**

1. A method for predicting the responsiveness of a subject to a non-surgical weight loss intervention, which comprises the step of determining, in an isolated test sample from the subject, the methylation status of:

   - one or more genes selected from the group consisting of NRP2, GSDMD, LOC102800447, NLRC5, GFRA1, LTBP3, and SPTLC2; or alternatively
   - the CpG position cg05387464; or alternatively
   - one or more genes selected from the group consisting of NRP2, GSDMD, LOC102800447, NLRC5, GFRA1, LTBP3, and SPTLC2; in combination with the CpG position cg05387464.

2. The method according to claim 1, wherein the subject suffers from overweight or obesity.

3. The method according to any of claims 1-2, wherein the subject is a child.

4. The method according to any of claims 1-3, which comprises determining the methylation status of:

   - NRP2 and NLRC5; or, alternatively
   - NRP2, NLRC5, GSDMD, and LOC102800447; or, alternatively
   - NRP2, NLRC5, GSDMD, LOC102800447, GFRA1, LTBP3, and SPTLC2; or, alternatively,
   - NRP2, NLRC5, GSDMD, LOC102800447, GFRA1, LTBP3, SPTLC2, and of the CpG position cg05387464.

5. The method according to any of claims 1-4, wherein the determining of the methylation status of the gene comprises determining the methylation status of one or more CpG positions in the gene.

6. The method according to claim 5, wherein the one or more CpG positions are selected from the group consisting of:

   - the CpG position cg14157435 of the NRP2 gene;
   - the CpG position cg16007266 of the NLRC5 gene;
   - the CpG position cg00036352 of the GSDMD gene;
   - the CpG position cg04057818 of the LOC102800447 gene;

- the CpG position cg08240913 of the GFRA1 gene;
- the CpG position cg23743554 of the LTBP3 gene; and
- the CpG position cg18872420 of the SPTLC2 gene.

7. The method according to any of the claims 1-6, wherein it is determined the methylation status of the CpG positions cg14157435, cg00036352, cg05387464, cg08240913, cg18872420, cg16007266, cg23743554, and cg04057818.

8. The method according to any of claims 1-7, which further comprises the step of determining the predicted differential body mass index z-score (DzBMI) with the formula (I):

$$DzBMI=-0.744+(-0.08m_1)+(-0.077m_2)+(-0.049m_3)+(-0.05m_4)+(-0.068m_5)+(-0.084m_6)$$
$$+(-0.058m_7)+(0.082m_8) \qquad (I)$$

wherein m1 is the log2 ratio of methylated probe intensity and unmethylated probe intensity for cg14157435;
wherein m2 is the log2 ratio of methylated probe intensity and unmethylated probe intensity for cg00036352;
wherein m3 is the log2 ratio of methylated probe intensity and unmethylated probe intensity for cg05387464;
wherein m4 is the log2 ratio of methylated probe intensity and unmethylated probe intensity for cg08240913;
wherein m5 is the log2 ratio of methylated probe intensity and unmethylated probe intensity for cg18872420;
wherein m6 is the log2 ratio of methylated probe intensity and unmethylated probe intensity for cg16007266;
wherein m7 is the log2 ratio of methylated probe intensity and unmethylated probe intensity for cg23743554;
wherein m8 is the log2 ratio of methylated probe intensity and unmethylated probe intensity for cg04057818;
wherein if the DzBMI is lower than or equal to -0.403, this is indicative that the individual will be a high-responder to the weight loss intervention; and
wherein if the DzBMI is higher than -0.403, this is indicative that the individual will be a low-responder to the weight loss intervention.

9. The method according to any of claims 1-8, wherein the weight loss intervention is selected from diet, physical exercise, and a combination thereof.

10. The method according to any of the claims 1-9, wherein the isolated test sample is blood.

11. Use of means for determining, in an isolated test sample, the methylation status of:

- one or more genes selected from the group consisting of NRP2, GSDMD, LOC102800447, NLRC5, GFRA1, LTBP3, and SPTLC2; or alternatively
- the CpG position cg05387464; or alternatively
- one or more genes selected from the group consisting of NRP2, GSDMD, LOC102800447, NLRC5, GFRA1, LTBP3, and SPTLC2, in combination with the CpG position cg05387464;

in a method for predicting the responsiveness of a subject to a weight loss intervention as defined in claim 1.

12. The use according to claim 11, wherein the means form part of a kit.

13. Use of:

- one or more genes selected from the group consisting of NRP2, GSDMD, LOC102800447, NLRC5, GFRA1, LTBP3, and SPTLC; or alternatively
- the CpG position cg05387464; or alternatively
- one or more genes selected from the group consisting of NRP2, GSDMD, LOC102800447, NLRC5, GFRA1, LTBP3, and SPTLC, in combination with the CpG position cg05387464;

as marker(s) for predicting the responsiveness of a subject to a weight loss intervention in an isolated test sample.

14. Use of one or more CpG positions selected from the group consisting of cg14157435, cg00036352, cg05387464, cg08240913, cg18872420, cg16007266, cg23743554, and cg04057818 as marker(s) for predicting the responsiveness of a subject to a weight loss intervention in an isolated test sample.

**15.** Use of a kit for predicting the responsiveness of a subject to a weight loss intervention, the kit comprising a solid support and means for determining the methylation status of:

- one or more genes selected from the group consisting of NRP2, GSDMD, LOC102800447, NLRC5, GFRA1, LTBP3, and SPTLC2; or alternatively
- the CpG position cg05387464; or alternatively
- one or more genes selected from the group consisting of NRP2, GSDMD, LOC102800447, NLRC5, GFRA1, LTBP3, and SPTLC2, in combination with the CpG position cg05387464.

Europäisches
Patentamt

European
Patent Office

Office européen
des brevets

## EUROPEAN SEARCH REPORT

Application Number

EP 18 38 2873

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ADRIANA MOLERES ET AL: "Differential DNA methylation patterns between high and low responders to a weight loss intervention in overweight or obese adolescents: the EVASYON study", THE FASEB JOURNAL, vol. 27, no. 6, 1 June 2013 (2013-06-01), pages 2504-2512, XP5555595, US ISSN: 0892-6638, DOI: 10.1096/fj.12-215566 * page 2505; figures 1,3,4 * | 1-15 | INV. C12Q1/6876 |
| A | LUCIA ARONICA ET AL: "A systematic review of studies of DNA methylation in the context of a weight loss intervention", EPIGENOMICS, vol. 9, no. 5, 1 May 2017 (2017-05-01), pages 769-787, XP055557977, United Kingdom ISSN: 1750-1911, DOI: 10.2217/epi-2016-0182 * the whole document * | 1-15 | |
| A | DIANJIANYI SUN ET AL: "Genetic, epigenetic and transcriptional variations at NFATC2IP locus with weight loss in response to diet interventions: The POUNDS Lost Trial", DIABETES, OBESITY AND METABOLISM, vol. 20, no. 9, 24 May 2018 (2018-05-24), pages 2298-2303, XP055558308, ISSN: 1462-8902, DOI: 10.1111/dom.13333 * the whole document * | 1-15 | |

TECHNICAL FIELDS
SEARCHED (IPC)

C12Q

-/--

The present search report has been drawn up for all claims

| Place of search | Date of completion of the search | Examiner |
|---|---|---|
| Munich | 21 February 2019 | Tilkorn, A |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.........................................................................

& : member of the same patent family, corresponding
    document

EPO FORM 1503 03.82 (P04C01)

Europäisches
Patentamt

European
Patent Office

Office européen
des brevets

## EUROPEAN SEARCH REPORT

Application Number

EP 18 38 2873

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LETICIA GONI ET AL: "Future Perspectives of Personalized Weight Loss Interventions Based on Nutrigenetic, Epigenetic, and Metagenomic Data", THE JOURNAL OF NUTRITION, vol. 146, no. 4, 1 April 2015 (2015-04-01), pages 905S-912S, XP055559229, US ISSN: 0022-3166, DOI: 10.3945/jn.115.218354 * the whole document * | 1-15 | |
| A | KHALIL ELGENDY ET AL: "Abstract", BRITISH JOURNAL OF NUTRITION, vol. 120, no. 9, 24 October 2018 (2018-10-24), pages 961-976, XP055559073, UK ISSN: 0007-1145, DOI: 10.1017/S000711451800243X * the whole document * | 1-15 | |
| A | LUIGI BOUCHARD ET AL: "Differential epigenomic and transcriptomic responses in subcutaneous adipose tissue between low and high responders to caloric restriction", THE AMERICAN JOURNAL OF CLINICAL NUTRITION, vol. 91, no. 2, 25 November 2009 (2009-11-25), pages 309-320, XP055559030, US ISSN: 0002-9165, DOI: 10.3945/ajcn.2009.28085 * the whole document * | 1-15 | TECHNICAL FIELDS SEARCHED (IPC) |

-/--

The present search report has been drawn up for all claims

| Place of search | Date of completion of the search | Examiner |
|---|---|---|
| Munich | 21 February 2019 | Tilkorn, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DELPHINE FRADIN ET AL: "Genome-Wide Methylation Analysis Identifies Specific Epigenetic Marks In Severely Obese Children", SCIENTIFIC REPORTS, vol. 7, no. 1, 7 April 2017 (2017-04-07), XP055558653, DOI: 10.1038/srep46311 * the whole document * | 1-15 | |

TECHNICAL FIELDS
SEARCHED (IPC)

The present search report has been drawn up for all claims

| Place of search | Date of completion of the search | Examiner |
|---|---|---|
| Munich | 21 February 2019 | Tilkorn, A |

EPO FORM 1503 03.82 (P04C01)

page 3 of 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MILAGRO FI et al.** A dual epigenomic approach for the search of obesity biomarkers: DNA methylation in relation to diet-induced weight loss. *FASEB J.,* 2011, vol. 25 (4), 1378-89
- **XU C.J. et al.** The emerging landscape of dynamic DNA methylation in early childhood. *BMC Genomics.,* 2017, vol. 18 (1), 25
- Lifestyle Intervention Decreases Urine Trimethylamine N-Oxide Levels in Prepubertal Children with Obesity. **LEAL-WITT MJ et al.** Obesity. Silver Spring, 2018, vol. 26, 1603-1610
- **MORRIS T.J. et al.** ChAMP: 450k Chip Analysis Methylation Pipeline. *Bioinformatics,* 2014, vol. 30 (3), 428-30

- **BUSATO F. et al.** Quantitative DNA Methylation Analysis at Single-Nucleotide Resolution by Pyrosequencing®. *Methods Mol Biol.,* 2018, vol. 1708, 427-445
- **MILAGRO F.I. et al.** A dual epigenomic approach for the search of obesity biomarkers: DNA methylation in relation to diet-induced weight loss. *FASEB J.,* 2011, vol. 25 (4), 1378-89
- **J. TYSON DEANGELIS et al.** An Overview of Epigenetic Assays. *Mol Biotechnol,* 2008, vol. 38 (2), 179-183
- **BURTIS C. A. et al.** Statistical Treatment of Reference Values. 2008